Europäisches Patentamt

**European Patent Office**

Office européen des brevets

(11) Publication number: **0 122 046**
**A1**

(12) **EUROPEAN PATENT APPLICATION**

(21) Application number: **84301597.5**

(22) Date of filing: **09.03.84**

(51) Int. Cl.³: **A 61 B 17/12**

(30) Priority: **11.03.83 US 474392**

(43) Date of publication of application: **17.10.84**
**Bulletin 84/42**

(84) Designated Contracting States: **DE GB**

(71) Applicant: **ETHICON INC., U.S. Route 22, Somerville New Jersey 08876 (US)**

(72) Inventor: **Mericle, Robert William, RD No. 3, Blossom Hill Road, Lebanon New Jersey (US)**

(74) Representative: **Jones, Alan John et al, CARPMAELS & RANSFORD 43 Bloomsbury Square, London, WC1A 2RA (GB)**

(54) **Absorbable fastening device with internal locking means.**

(57) A non-metallic, bio-compatible, sterile, surgical device (20) having a pair of leg members (21, 22) joined at their proximal ends (23). The device has an interlocking latch means (26, 27) adjacent the joined proximal ends.

Absorbable Fastening Devices With Internal Locking Means

Background of the Invention

The present invention relates to improved surgical devices made from polymeric materials and more particularly to tissue fastening devices such as staples, clips and the like. Many of the present day fastening devices used in medicine and surgery are made from metals such as stainless steel or tantalum. For example, skin staples have been used for some time now with these staples made from stainless steel, tantalum and the like. Similarily, hemostatic or ligating clips have also been made from similar metals. Though these metal devices have gained considerable acceptance in the case of skin staples, they must be removed which, in many instances, can be painful and requires a further operation or manipulation of the staple. When the metal fasteners are used internally they are left in the patient and this may be disruptive in subsequent diagnostic imaging procedures such as x-rays, CAT scanning and the like. Because of the interference that such metal provides, they are not desirable in all instances. The same is true for the metal ligating or hemostatic clips. As substitutes and to replace these metallic surgical devices, there have been recently developed a number of bio-compatible, non-metallic, surgical devices. Examples of such non-metallic surgical devices are described in EP-A-0 073 655, GB-A-2 054 026, GB-A-2 054 027, and U.S. patent application No. 349 443.

The surgical devices, whether they be skin staples, internal fasteners, ligating clips or the like, should be easy to manipulate. That is, they should be readily held with

0122046

assurance by whatever instrument is being used to apply them. They should be held in such a manner that the instrument may be passed from nurse to surgeon, the instrument inserted, moved around and otherwise manipulated. without losing the surgical device from the instrument. Especially with internal surgical devices, bulk is important. The less material placed within the human cavity the less chance for adverse reactions. Not only should the device be non-bulky and as small as possible, but it is preferred it be as smooth and streamlined as possible. The smoother and more streamlined the device, the less trauma is caused and the less chance the device may be dislodged as it is placed. The smoother and more streamlined the device, the less chance there is that the surgeon or nurse will catch a corner of a sponge or piece of gauze on the device when they are cleaning the surgical site or trying to maintain that surgical site dry and free of blood.

Along with these properties, the device, whether it be a fastener or a clip or a skin staple, must fasten and perform its function of either holding tissue together or closing the vessel or the like in a secure manner.

The present invention provides a novel clip that is easy to manipulate. Furthermore, the new clip may be manipulated with great assurance. The new clip is non-bulky and has smooth, streamlined curves and surfaces throughout its outer configuration; that is, those surfaces that will come into contact with adjacent tissue or may be contacted by sponges or gauzes used in and around the wound. Furthermore, the new clip has a secure locking mechanism which does not disrupt or interfere with its smooth contoured curved surfaces.

ETH-572

-3-                          0122046

## Summary Of The Present Invention

The new surgical device of the present invention is non-metallic and bio-compatible. The device has an open position and a closed position. The device comprises a pair of leg members having inner surfaces disposed towards one another. The leg members are joined at their proximal ends by a hinge means. In certain embodiments of the present invention, the hinge means is resilient. The distal ends of the leg members include means for engaging tissue. In some embodiments of the present invention, the tissue engaging means are the surfaces of the legs which are used to clamp about a blood vessel and close the blood vessel. In other embodiments of the present invention, the distal ends of the leg members may include sharpened, penetrating portions which actually penetrate and are inserted into tissue to hold tissue together. My new device includes an interlocking latch means disposed immediately adjacent the hinge means and disposed towards the distal ends of the leg members. The latch means comprises an engaging member which is disposed inwardly from the inner surface of the first leg member. This engaging surface is disposed in a direction towards the inner-surface of the second leg member. The latch means includes locking means disposed from the inner surface of the second leg member. When the leg members are urged towards one another to move the clip from an open position to a closed position, the engaging means engages the locking means to secure the device in the closed position. In certain embodiments, the engaging means and the locking means may be oppositely disposed hooking members; that is, with one hook undercutting and hooking with an oppositely disposed hook. In other embodiments of the present invention, the engaging means may include a male member as the locking means with a female portion in the opposite leg member with the two engaging when the clip is closed.

-572

In other embodiments of the device of the present invention, the engaging means may include stepped or recessed portions in one leg member and complementary stepped or raised portions in the opposite leg member which interengage with each other when the clip is in the closed position.

Brief Description Of The Drawings

Figure 1 is a perspective view of a tissue fastening device of the present invention with the device in the open position;

Figure 2 is a perspective view of the device of Figure 1 in a closed and locked position;

Figure 3 is a perspective view of a hemostatic clip of the present invention with the clip depicted in the open position;

Figure 4 is a perspective view of the clip of Figure 3 in the closed and locked position;

Figure 5 is a view in perspective of another embodiment of a tissue fastening device in accordance with the present invention with the device shown in the open position;

Figure 6 is a perspective view of the device of Figure 5 in a closed and locked position;

Figure 7 is a view in perspective of a skin fastening device of the present invention with the device depicted in the open position;

Figure 8 is a perspective view of the device of Figure 7 with the device in the closed and locked position;

Figure 9 is a plan view of one type of instrument used for applying the surgical devices of the present invention; and

Figure 10 is a plan view of another type of instrument for applying a multiplicity of the devices of the present invention.

## Detailed Description Of The Invention

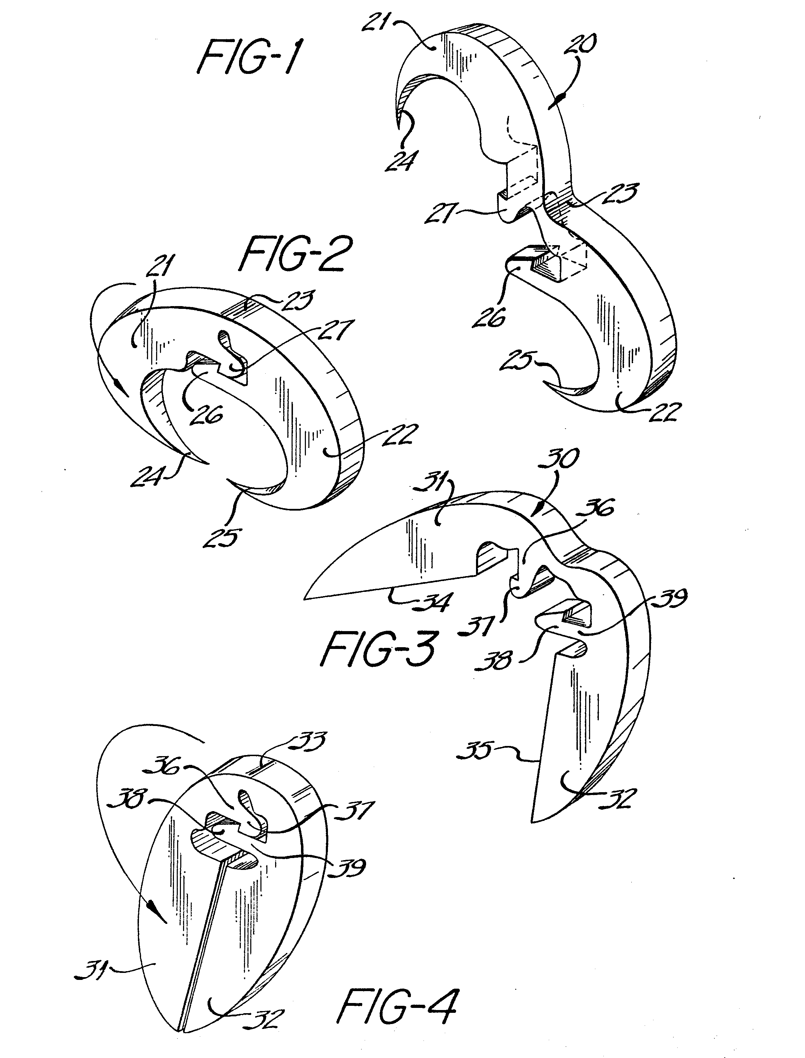

Referring to the drawings, in Figures 1 and 2 there is shown a tissue fastening device 20 of the present invention. The device comprises a pair of leg members 21 and 22. The leg members of this embodiment are connected at a hinge portion 23 and are movable or closable about that hinge portion. The distal ends of the leg members terminate in tissue penetrating portions 24 and 25. These are sharpened pointed ends which are adapted to penetrate tissue. Adjacent the hinge and positioned towards the distal ends is a latch means. The latch means comprises oppositely facing hook members 26 and 27. The first hook member 26 or engaging means is disposed on one leg member and faces the opposite leg member with the hook portion disposed toward the hinge. The hook member 27 of the other leg member or the locking means is disposed on the other leg member and faces the first leg member with the hook portion disposed towards the distal end of the leg members. When the legs are rotated about the hinge means, the two hooks interengage each other to lock and secure the leg members together and the device in the closed position. Hence, in operation, it is a simple manipulation to insert the distal pointed end of one of the leg members into one piece of tissue, allow the other distal member to grasp adjacent tissue and then rotate the two leg members towards each other about the hinge to bring the tissue together and allow the device to lock

-572

into position securing the tissue between the distal ends of the leg members. As may be seen in the drawings, the device has smooth curves and when in its closed position has a generally oval or eliptical shape so that irritation and traumatic effects of surrounding tissue are kept to a minimum and there are no sharp protruding or rough edges that might be engaged by a sponge utilized by a surgeon or a nurse when cleaning that area being worked on during the procedure. The locking means itself is hidden and secured so that the tendency to dislodge or disrupt the fastener is kept to a minimum.

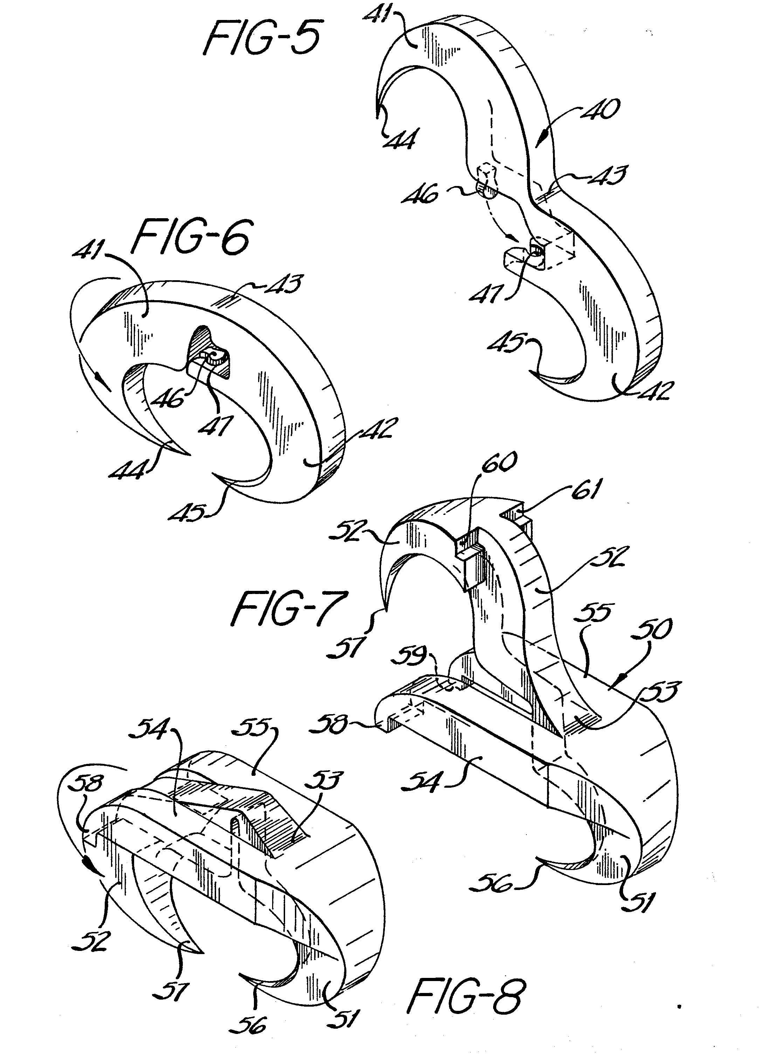

In Figures 3 and 4 there is shown a hemostatic clip 30 of the present invention. In this embodiment, the clip comprises a pair of leg members 31 and 32 connected at their proximal ends by a hinge means 33. The leg members have innersurfaces 34 and 35 facing each other. These innersurfaces are vessel clamping surfaces. Adjacent the vessel clamping surface on one leg member 31 and disposed towards the distal end of that leg member is an engaging means which is a hook 36 having the hook portion 37 disposed towards the distal end of the leg member. Adjacent the hinge means on the opposite leg member 32 is a locking means which again is a hook 38 having the hook portion 39 disposed towards the proximal end of the leg member. In use, the vessel clamping surfaces are placed on opposite sides of the vessel to be closed and the vessel clamping surfaces urged towards one another until the two members engage one another and lock the clip in its closed position thus closing the vessel placed between the vessel clamping surfaces. Again, as may be seen, the clip has a very smoothly contoured outer surface so that irritation and trauma of surrounding tissue is kept to a minimum and the chances of removal or disruption of the clip by a nurse or surgeon cleaning the wound or the surgical site is kept at a minimum. Due to the plastic flex properties

0122046

of some polymeric materials, it may be desirable to alter the design so that the tips of the clamping surfaces meet prior to locking. Additional closing force may then be applied to bend the leg members and pre-load the clamp surfaces in the final locked position. It should be pointed out that even if the hinge means 33 breaks after the clip has been placed, the clip will remain in the closed locked position by virtue of the latch means. This is a considerable advantage especially when making these clips of various polymeric materials which are brittle and tend to fracture. It is also a considerable advantage with clips made from absorbable polymeric materials. With the absorbable polymers the higher stressed areas tend to be absorbed first and, hence, are the first area to break. This is the hinge area in a hemostatic clip. This configuration of latch means is a major improvement in insuring that the polymeric types of surgical devices remain secure in their closed positon.

Referring to Figures 5 and 6 there is shown another embodiment of a tissue fastening device of the present invention. In this embodiment, the device 40 comprises a pair of leg members 41 and 42 joined at their proximal ends by a resilient hinge 43. The distal end of each leg member terminates in a pointed sharp tissue penetrating means 44 and 45. On one of the leg members adjacent the hinge and disposed towards the other leg member is a male engaging means 46. Disposed on the other leg member adjacent the hinge is a locking means which is a slot 47 which provides an interference fit for the male member. On closing the device about the tissue to be joined, one of the tissue penetrating means is inserted into one portion of the tissue and the opposite tissue penetrating means is inserted into adjacent tissue. The two leg members are urged together and the male member is pushed into the slot

0122046

past the narrowed opening and is held therein by the narrowed opening.

In Figures 7 and 8 there is shown another fastener. In Figure 7 the fastener 50 is shown in its open position. The fastener comprises a pair of leg members 51 and 52 connected at a hinge means. In this embodiment, one leg member 51 has two rigid portions 54 and 55 extending on opposite sides of the other leg member 52. The distal ends of the leg members terminate in skin or tissue penetrating means 56 and 57; that is, sharpened points. The rigid portions extending beyond the leg member 51 terminate in downwardly extending raised lip areas 58 and 59. The opposite leg member 52 which is disposed between the two rigid portions has two depressed lip portions 60 and 61 spaced from the distal end of the leg member. As is more clearly shown in Figure 8, when the two leg members are urged towards one another, the depressed lip portions 60 and 61 are engaged by the raised lip areas 58 and 59 of the opposite leg member to lock the two members together.

As previously mentioned, the hinge means of the device of the present invention may be resilient or non-resilient and, in fact, even if they are so non-resilient that upon closing they crack or break or weaken, it does not effect the performance of the device. Once the devices of the present invention are closed, they unexpectedly remain secured in the closed position even if the hinge subsequently breaks.

As may be appreciated from the previous description, the distal ends of the leg members which are adapted to perform a surgical function may either be flat vessel clamping surfaces so that the device may be used as a ligating or hemostatic clip or they may be sharp pointed penetrating devices which would be used to penetrate and secure tissue together. If penetrating members are used, the spacing of the penetrating members when the device is closed may be different depending on what tissue is to be joined. Also, as can be appreciated, the latch means may include various configurations provided it is disposed within the confines of the device itself. Male and female latching members, hook latch members, and the like, may be used.

The devices of the present invention may be constructed in various sizes according to their intended function. Hemostatic clips are typically less than 6 millimeters in length, about 1.5 millimeters in width and have a vessel clamping surface of about 2 millimeters in length. The dimensions of the clip may be reduced by about 50% for certain applications in microsurgery. Larger clips, for special hemostatic applications and other functions such as closures for oviducts or vas deferens may have dimensions of about double those of a hemostatic clip. Tissue fastening devices are typically less than about 10 millimeters in length and about 6 millimeters in width and have tissue penetrating portions of 4 millimeters. These dimensions may also be reduced by about 50% for certain applications in microsurgery and may be increased for various other uses.

The devices of the present invention are most conveniently molded of biologically acceptable plastic materials which may be absorbable or non-absorbable. Preferred absorbable polymers include homopolymers and copolymers of glycolide and lactide and polydioxanone. Preferred non-absorbable polymers include nylon, polyester, and polypropylene. All these materials have been demonstrated to be biologically acceptable when used as sutures or other implantable medical devices. The devices may be also cast or machined from polymeric material as desired.

-572

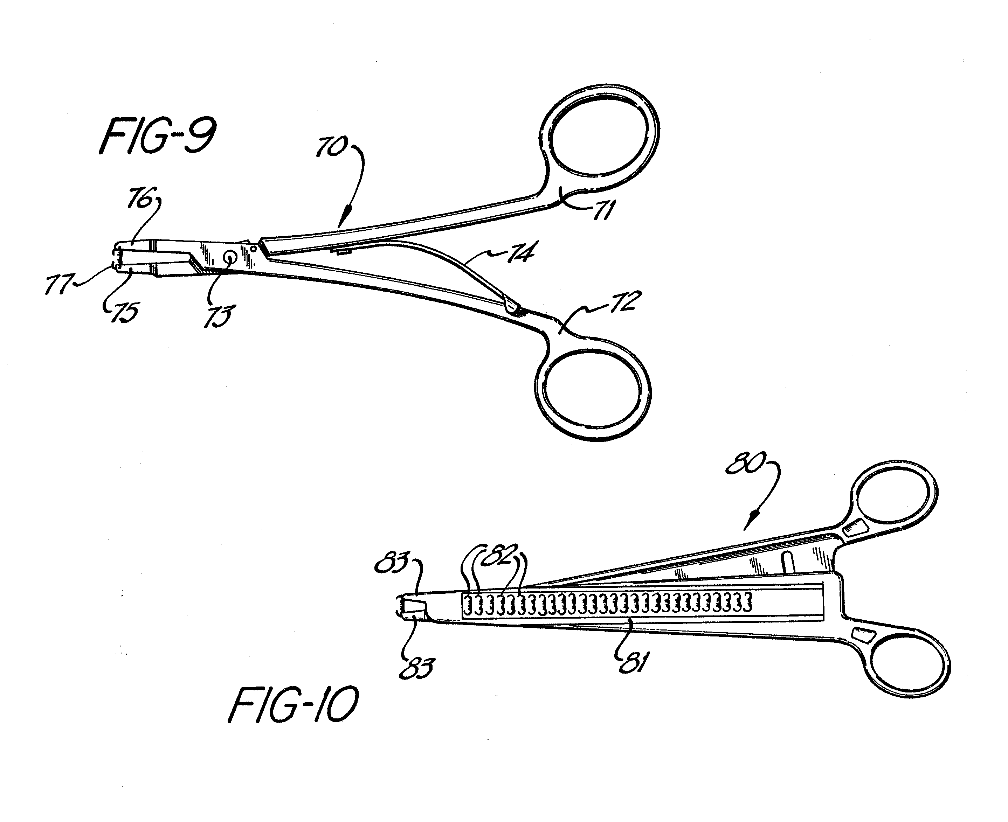

In utilizing the devices of the present invention, the instrument with which the device is placed may be a single application instrument such as that shown in Figure 9 or a multiple application instrument such as that shown in Figure 10.

Referring to Figure 9 there is shown a forceps type instrument 70 comprising two handle members 71 and 72 crossing at a hinge point 73 and maintained in a normally open position by a spring 74. The handle 71 extends beyond the hinge, forming jaw member 75, while the extension of the other handle 72 forms the opposite jaw member 76. A device 77 of the present invention may be picked up in these jaw members with the device in the open position. The device may then be placed at the desired surgical site and the device closed by urging the handles towards one another thus closing the device and locking it via its latch means to perform its appropriate function. If desired, the instrument may be a multiple applying device such as that shown in Figure 10. In this embodiment, the instrument 80 includes a cartridge 81 containing a multiplicity of the surgical devices 82 of the present invention. The mechanical features in the instrument present individual devices to the jaws 83 of the instrument and allow the jaws to close and, hence, close and lock the device in place. The mechanical device then, upon the reopening of the jaws, presents a new device to those jaws for subsequent closure. The specific mechanical techniques for accomplishing this are not being discussed as they do not form any portion of the present invention.

The configuration of the device of the present invention is important and very beneficial when making devices of absorbable polymers. In making devices from absorbable polymers, it is generally the case that the hinge area is stressed the greatest during the molding and manufacturing

ETH-572

of the device. The highly stressed hinge area tends to be absorbed or weakened before the remainder of the clip is absorbed and weakened. This fact does not alter the efficacy of the new device because even if the hinge cracks, breaks, or is weakened, the device is held and locked in its closed position. In prior art devices, the hinge is generally under tension when the clip is closed and hence is being forced apart so when the hinge weakens the device can open. Moving the hinge to this new position alters the secondary function of holding the device closed to the function of a fulcrum under compression.

Another important aspect to placing the latch means adjacent the hinge means is that it reduces transverse movement of the leg members. By placing the latch means adjacent the hinge, greater divergance of the legs is allowed before the latch unlocks. The new devices are self interlocking and have reduced unlocking because of transverse movement of the leg members.

The smooth, continuous curved configurations of the new devices not only is important in providing improved performance in the surgical procedure involved but also allow the devices to be readily molded or otherwise produced simply and economically.

Depending on the material used to make the new device, the device may be sterilized by the various well known sterilization techniques; such as, heat, radiation, ethyleneoxide, and the like.

Having now described the invention and certain specific embodiments thereof, it would be readily apparent to one skilled in the art that many variations and modifications may be made to the present invention without departing from the spirit and scope thereof.

CH-572

CLAIMS

1. A non-metallic, bio-compatible sterile, surgical device operable during a surgical procedure to be positioned in a desired location and secured therein to prevent involuntarily dislodgment from said location, said device comprising: a pair of leg members adapted at their distal ends to perform a surgical function, said leg members being joined together at their proximal ends, an integral, interlocking latch means immediately adjacent the joined proximal ends and disposed towards the distal ends of the said leg members for securing the leg members together when said leg members are urged towards one another to position and secure said device in the desired location, said latch means being disposed totally within the confines of the leg members whereby when the device is secured in a desired location, the latch means is protected to prevent voluntarily dislodgment.

2. A device according to claim 1, wherein said latch means comprises an engaging member disposed inwardly from the inner surface of one leg member towards the inner surface of the other leg member, and locking means disposed on the inner surface of the other leg member adapted to co-operate with said engaging member.

3. A non-metallic, bio-compatible, sterile, surgical device having an open position and a closed position, said device comprising: a pair of leg members having vessel clamping inner surfaces disposed towards one another, said leg members joined at their proximal ends in a hinge means, an integral, interlocking latch means immediately adjacent the hinge means disposed towards the distal ends of said leg members, said latch means comprising an engaging member disposed inwardly from the inner surface of one leg member towards the inner surface of the other leg member and locking means disposed from the inner surface of the other leg member whereby when the inner surfaces of said leg members are urged towards one another said engaging means engages said locking means to secure said device in a desired position.

4. A non-metallic, bio-compatible, sterile, surgical device having an open position and a closed position, said device comprising: a pair of leg members joined at their proximal ends in a hinge means, the distal ends of said leg member terminating in tissue penetrating means, interlocking latch means disposed immediately adjacent said hinge means and disposed towards the tissue penetrating means, said latch means comprising an engaging member disposed inwardly from the inner surface of one leg member towards the inner surface of the other leg member and locking means disposed in the inner surface of the other leg member adapted to cooperate with said engaging member whereby when the leg members are urged towards one another, said engaging means engages said locking means to secure said device in the closed position.

5.— A device according to claim 4, wherein the tissue penetrating means comprise sharpened points.

6. A device according to claim 5, wherein the sharpened points abut one another when the device is in the closed position.

7. A device according to any one of claims 1 to 6, wherein the leg members are joined at their proximal ends by a resilient hinge portion.

0122046

8. A device according to any one of claims 2 to 4 or any claim dependent thereon wherein the engaging member is a hook extending inwardly from the inner surface of one leg member and the locking means is a complementary hook extending inwardly from the inner surface of the other leg member.

9. A device according to any one of claims 2 to 4, or any claim dependent thereon wherein the engaging member is a protrusion extending inwardly from the inner surface of one leg member and the locking means is a slot disposed in the inner surface of the other leg member.

10. A device according to any one of claims 1 to 9 made from an absorbable polymeric material.

11. A device according to claim 10, made from polydioxanone.

12. A device according to any one of claims 1 to 9, made from a non-absorbable polymeric material.

FIG-1

21 · 20 · 24 · 27 · 23 · 26 · 25 · 22

FIG-2

21 · 23 · 27 · 26 · 22 · 24 · 25

FIG-3

31 · 30 · 36 · 34 · 31 · 39 · 38 · 35 · 32

FIG-4

36 · 33 · 38 · 31 · 39 · 31 · 32

FIG-5

FIG-6

FIG-7

FIG-8

FIG-9

76
77
75
73
70
71
74
72

FIG-IO

83
82
83
80
81

3/3

0122046

# 0122046

## EUROPEAN SEARCH REPORT

European Patent Office

Application number

EP ·84 30 1597

### DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| Y | GB-A-1 020 035 (POSSE)<br><br>* Whole document *<br>--- | 1-3,10-12,4-7 | A 61 B 17/12 |
| Y | EP-A-0 072 232 (ETHICON)<br><br>* Claims; figures *<br>--- | 1-3,10-12 | |
| Y | GB-A- 293 301 (KIRURGISKA)<br>* Whole document *<br>--- | 4-7 | |
| D,A | EP-A-0 073 655 (ETHICON)<br>* Figures *<br>--- | 8,9 | |
| P,X | EP-A-0 095 249 (ETHICON)<br>* Whole document *<br><br>----- | 1-12 | |

**TECHNICAL FIELDS SEARCHED (Int. Cl. ³)**

A 61 B
A 61 F

The present search report has been drawn up for all claims

| Place of search | Date of completion of the search | Examiner |
|---|---|---|
| THE HAGUE | 18-06-1984 | LOWE D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82